(19) Europäisches Patentamt
European Patent Office
Office européen des brevets

(11) Veröffentlichungsnummer: **0 002 182**
**B1**

(12) **EUROPÄISCHE PATENTSCHRIFT**

(45) Veröffentlichungstag der Patentschrift: **08.04.81**

(21) Anmeldenummer: **78101084.8**

(22) Anmeldetag: **06.10.78**

(51) Int. Cl.³: **C 07 D 209/48,**
C 07 C 149/43,
C 07 C 147/13,
C 07 C 117/08 //C08F8/30

# (54) Bis-Azidophthalsäurederivate und Verfahren zu ihrer Herstellung.

(30) Priorität: **14.10.77 CH 12579/77**

(43) Veröffentlichungstag der Anmeldung:
**13.06.79 Patentblatt 79/12**

(45) Bekanntmachung des Hinweises auf die Patenterteilung:
**08.04.81 Patentblatt 81/14**

(84) Benannte Vertragsstaaten:
**CH DE FR GB NL SE**

(56) Entgegenhaltungen:
**GB - A - 1 309 959**
**US - A - 2 852 379**

(73) Patentinhaber: **CIBA-GEIGY AG**
**Patentabteilung Postfach**
**CH-4002 Basel (CH)**

(72) Erfinder: **Kvita, Vratislav, Dr.**
**St. Jakobstrasse 2**
**CH-4132 Muttenz (CH)**
Erfinder: **Zweifel, Hans, Dr.**
**Lothringerstrasse 93**
**CH-4056 Basel (CH)**
Erfinder: **Greber, Gerd, Prof. Dr.**
**Anzengruberstrasse 11**
**AT-2540 Bad Vöslau (AT)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 002 182 B1

## Bis-Azidophthalsäurederivate und Verfahren zu ihrer Herstellung

Die vorliegende Erfindung betrifft neue Bis-Azidophthalsäurederivate und Verfahren zu deren Herstellung. Die erfindungsgemässen Bis-Azidophthalsäurederivate eignen sich zur Herstellung von unter der Einwirkung von Licht härtbaren (vernetzbaren) Stoffgemischen.

Aus der Literatur ist bekannt, dass sich Azido-Phenylderivate, wie 4,4'-Diazidostilben, das Dinatriumsalz von 4,4'-Diazidostilben-2,2'-disulfonsäure, 4,4'-Diazidobenzophenon, 4,4'-Diazidochalkon, 2,6-Di-(4'-Azidobenzal)-cyclohexanon, 2,6-Di-(4'-Azidobenzal)-4-methylcyclohexanon, 4,4'-Diazidobenzalaceton und Bis-(4'-Azidocinnamyliden)-cyclopentanon, als Sensibilisatoren für Polymere für phototechnische Zwecke, z.B. sog. Photoresists, oder als lichtempfindliche Komponente in Kolloidschichten, wie Gelatine oder Kasein, zur Erzeugung von sogenannten Gerbbildern in der Photographie oder bei Verfahren in der Reproduktionstechnik eignen (vgl. z.B. US Patentschriften 2.852.379, 2.940.853 und 3.749.713, deutsche Patentschrift 752.852, russische Patentschrift 503.855 und britische Patentschrift 892.811).

Diese vorbekannten Azido-Phenylderivate sind insofern nachteilig, als die thermische Stabilität und die Oxidationsbeständigkeit derselben oder von Gemischen, die dieselben enthalten, zu wünschen übrig lassen.

In der DE—AS 2 000 623 ist ein photographisches Verfahren zur Herstellung von Bildern beschrieben, wobei lichtempfindliche, cyclische, aromatische Dicarbonsäureimide, welche keine Azidogruppen enthalten, zur Anwendung kommen. Bei diesen Imiden handelt es sich um photochrome Systeme; d.h. es entsteht ein Farbstoff und es resultiert ein Bild, für dessen Entstehung aber sehr spezielle Stoffe (z.B. $TiO_2$) zugegen sein müssen. Es treten nur geringe Löslichkeitsunterschiede zwischen den belichteten und unbelichteten Teilen der Schicht auf. Von einer regelrechten Vernetzung der Masse infolge Belichtung kann nicht die Rede sein und deshalb sind derartige lichtempfindliche Massen für Photoresistsysteme ungeeignet.

Aufgabe der Erfindung war daher die Bereitstellung von neuen Substanzen mit verbesserter thermischer Stabilität und Oxidationsbeständigkeit bei gleich guter Lichtempfindlichkeit wie die der oben angeführten bekannten Substanzen. Weiter sollen diese neuen Substanzen bei einer Belichtung zu einer echten Vernetzung und nicht nur zu geringen Löslichkeitsunterschieden führen.

Die neuen Bis-Azidophthalsäurederivate entsprechen der Formel Ia oder Ib

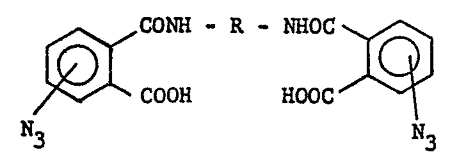

(Ia)

oder

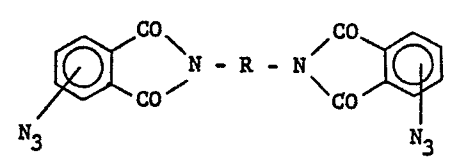

(Ib)

worin

R unsubstituiertes oder substituiertes Alkylen mit 2—18 C-Atomen, unsubstituiertes oder substituiertes Phenylen, Naphthylen, Biphenylen, Cyclohexylen, unsubstituiertes oder substituiertes Dicylohexylenmethan oder eine unsubstituierte oder substituierte Gruppierung

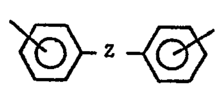

und

Z —O—, —S—, —$SO_2$—, —$CH_2$—, —CO—, 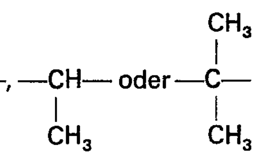

bedeuten.

Eine Vorzugsform der Erfindung stellen solche Verbindungen der Formeln Ia und Ib dar, worin R unsubstituiertes oder durch eine oder zwei Phenylgruppen, Cycloalkylgruppen mit 5—8 C-Atomen oder Aralkylgruppen mit 7 oder 8 C-Atomen, substituiertes Alkylen mit 2—12 C-Atomen, unsubstituiertes oder pro Ring durch eine Alkylgruppe mit 1—4 C-Atomen, eine —OH—, —COO—$M^+$— oder —$SO_3$—$M^+$—Gruppe substituiertes Phenylen, Naphthylen, Biphenylen, Cyclohexylen, Dicyclohexylenmethan oder

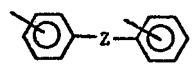

darstellt, Z die unter Formel Ia bzw. Ib angegebene Bedeutung hat, $M^+$ das Wasserstoffion, ein Alkalimetallkation, das Pyridiniumkation oder

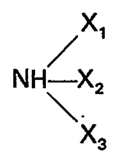
,

$X_1$ und $X_2$ unabhängig voneinander Wasserstoff oder Alkyl mit 1—12 C-Atomen und $X_3$ Wasserstoff, Alkyl mit 1—12 C-Atomen oder Benzyl bedeuten.

Beispiele der Alkylengruppen R sind: die 1,2-Aethylen-1,3- oder 1,2-Propylen-, 1,4- oder 1,3-Butylen-, Pentamethylen-, Hexamethylen-, 2-Methyl-4-dimethylhexylen, 2-Dimethyl-4-methylhexylen-, 1,10-dicyclohexylen- und 1,10-Dicyclooctylendecan-, 1-10-Diisoproplyendecan-, 1,1,10,10-Tetramethyldecylen-, 1,10-Diäthyl, 1,10-dimethyldecylen-, Octamethylen-, Decamethylen-, und Dodecamethylengruppe.

Durch R dargestellte Phenylen-, Naphthylen-, Biphenylen-, Cyclohexylen- und Dicyclohexylen-, methangruppen und Gruppierungen R

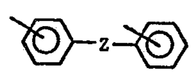

können ebenfalls substituiert sein, z.B. durch Alkylgruppen mit 1—4 C-Atomen, —OH—, —$COO^-M^+$— oder —$SO_3^-M^+$-Gruppen, wobei $M^+$ das Wasserstoffion, ein Alkalimetallkation, das Pyridiniumkation, oder

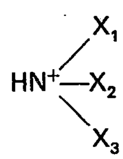

$X_1$ und $X_2$ unabhängig voneinander Wasserstoff oder Alkyl mit 1—12 C-Atomen und $X_3$ Wasserstoff, Alkyl mit 1—12 C-Atomen oder Benzyl bedeuten. Alkylgruppen $X_1$, $X_2$ und $X_3$ können geradkettig oder verzweigt sein. Vorzugsweise handelt es sich um geradkettige Alkylgruppen mit 1—4 C-Atomen. Die genannten Gruppen R können an jedem Ring mehrere Substituenten der erwähnten Art aufweisen, sind aber mit Vorteil pro Ring nur durch eine der genannten Gruppen substituiert. Als Alkylsubstituenten kommen besonders Methyl und Aethyl in Betracht. Stellt $M^+$ eine Gruppierung

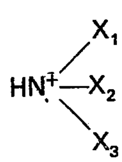

dar, so bedeuten $X_1$ und $X_2$ bevorzugt Alkylgruppen mit 1—4 C-Atomen und $X_3$ eine Alkylgruppe mit 1—4 C-Atomen oder die Benzylgruppe.

Bevorzugte —$COO^-M^+$— oder —$SO_3^-M^+$—Substituenten sind solche, worin $R^+$ das Wasserstoffion, ein Alkalimetallkation, ein Benzyldialkyl- oder Trialkylammoniumkation mit je 1—4 C-Atomen in den Alkylteilen darstellt. Besonders bevorzugt bedeutet $M^+$ das Wasserstoffion, das Natrium- oder Kaliumkation.

Phenylen-, Naphthylen-, Biphenylen-, Cyclohexylen- und Dicyclohexylenmethangruppen oder Gruppen

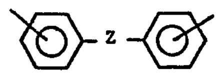

sind bevorzugt unsubstituiert. Bevorzugte Brückenglieder Z sind —O—, —$SO_2$— und —$CH_2$—.

Die $N_3$-Gruppen sind bevorzugt je in ortho-Stellung zu einer Carbonyl- bzw. Carboxylgruppe oder Carbonamidgruppe an den Benzolring gebunden.

Besonders bevorzugt sind Verbidnungen der Formel Ia und Verbindungen der Formel Ib, worin R unsubstituiertes geradkettiges oder verzweigtes Alkylen mit 2—12 und besonders 2—10 C-Atomen, unsubstituiertes Phenylen, Biphenylen oder einen unsubstituierten Diphenylenäther-, Diphenylenmethan- oder Diphenylensulfonrest oder den 3,3'-Dimethyl-dicyclohexylenmethanrest bedeuten.

Besonders bevorzugte erfindungsgemässe Substanzen sind die Verbindungen der Formel VI

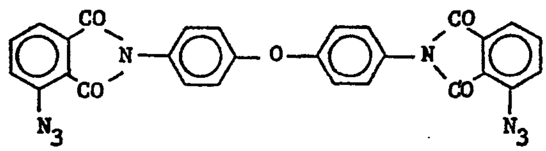

(VI)

und die Verbindungen der Formel VII

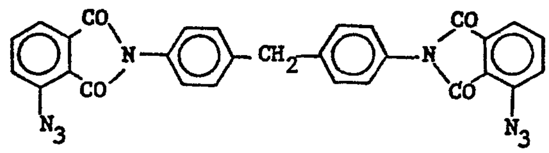

(VII)

und die Verbindungen der Formel VIII

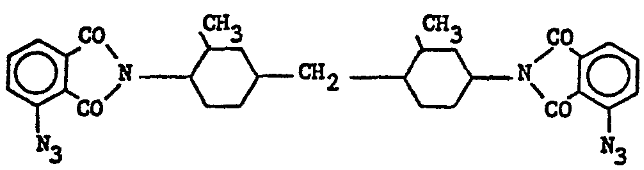

Die Verbindungen der Formel Ia und Ib können dadurch hergestellt werden, dass man eine Verbindung der Formel II

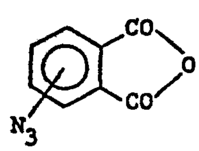

(II)

in einem Mol-Verhältnis von mindestens 1:2 mit einem Diamin der Formel III

$$H_2N—R—NH_2 \quad (III)$$

zu einer Verbindung der Formel Ia

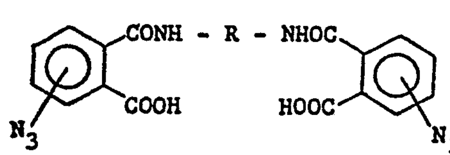

(Ia)

umsetzt, wobei R die unter Formel Ia bzw. Ib angegebene Bedeutung hat, und die Verbindung der Formel Ia gegebenenfalls anschliessend cyclisiert.

Die Umsetzung der Anhydride der Formel II mit den Diaminen der Formel III wird zweckmässig in organischem Medium vorgenommen, wobei je nach Art der Reaktionskomponenten bei Temperaturen zwischen etwa 0°C und 120°C gearbeitet wird.

Zweckmässig setzt man das Anhydrid der Formel II in stöchiometrischer Menge oder in einem

leichten Ueberschuss über das Diamin der Formel III ein, z.B. in einem bis zu etwa 20%-igen molaren Ueberschuss.

Als organische Lösungsmittel kommen vor allem aprotische Lösungsmittel in Betracht. Beispiel geeigneter aprotischer organischer Lösungsmittel sind:

gegebenenfalls chlorierte aliphatische oder aromatische Kohlenwasserstoffe, wie Benzol, Toluol, Methylenchlorid, chloroform, Tetrachlorkohlenstoff, 1,1,2-Trichloräthan, 1,2-Dichloräthylen und Chlorbenzol;

aliphatische und cycloaliphatische Ketone, wie Aceton, Methyläthylketon, Cyclopentanon und Cyclohexanon;

cyclische Aether, wie Tetrahydrofuran, Tetrahydropyran und Dioxan;

cyclische Amide, wie N-Methyl-2-pyrrolidon, N-Acetyl-2-pyrrolidon; N-Methyl-ε-caprolactam;

N,N-Dialkylamide von aliphatischen Monocarbonsäuren mit 1—3 C-Atomen im Säureteil, wie N,N-Dimethylformamid, N,N-Dimethylacetamid, N,N-Dimethylmethoxyacetamid;

Alkylester von aliphatischen Monocarbonsäuren mit insgesamt 2—6 Kohlenstoffatomen. wie Ameisen- oder Essigsäuremethyl, -äthyl- und n-butylester;

Hexamethylphosphorsäuretriamid (Hexametapol);

N,N,N',N'-Tetramethylharnstoff;

Tetrahydrothiophendioxid (Sulfolan);

Dialkylsulfoxide, wie Dimethyl- oder Diäthylsulfoxid.

Es können auch Gemische derartiger Lösungsmittel eingesetzt werden.

Die Cyclisierung der Verbindungen der Formel Ia kann auf an sich bekannte Weise chemisch, d.h. unter Zusatz an sich bekannter Dehydratisiertungsmittel, vorgenommen werden. Je nach Art der Reaktionskomponenten, der Reaktionsbedingungen und des eingesetzten Lösungsmittels kann die Cyclisierung, besonders bei erhöhten Temperaturen, auch ohne Zusatz eines Dehydratisierungsmittels erfolgen, wobei das gebildete Wasser zweckmässig azeotrop entfernt wird.

Die Cyclisierung wird im allgemeinen bei Temperaturen zwischen etwa 40 und 120°C, bevorzugt 70—90°C, in Gegenwart von Dehydratisierungsmitteln und gegebenenfalls in Gegenwart eines aprotischen organischen Lösungsmittels durchgeführt. Als Dehydratisiertungsmittel kommen vor allem Anhydride von gegebenenfalls durch Halogenatome oder Alkylgruppen substituierten aliphatischen Monocarbonsäuren mit 2—5 C-Atomen in Betracht, wie Essigsäure-, Propionsäure-, Buttersäure- und Valeriansäureanhydrid, Trichlor- und Trimethylessigsäureanhydrid. Bevorzugtes Dehydratisiertungsmittel ist Essigsäureanhydrid.

Die Verbindungen der Formel Ib können nach einem bevorzugten Verfahren auf besonders einfache Weise auch direkt dadurch hergestellt werden, dass man eine Verbindung der Formel IV

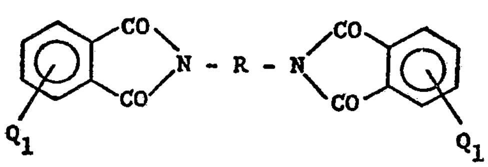

(IV)

worin
R die unter Formel Ia bzw. Ib angegebene Bedeutung hat und
$Q_1$ ein Halogenatom, wie Chlor, Fluor oder Brom, oder die Nitrogruppe bedeutet,
in einem inerten organischen Lösungsmittel bei einer Temperatur zwischen etwa 0 und 120°C, bevorzugt zwischen etwa 70 und 100°C, mit einem Azid der Formel V

$$M_1^{n+} (N_3^-)_n \qquad (V)$$

umsetzt, worin
n die Zahl 1 oder 2 und
$M_1$ ein Alkalimetall-, Erdalkalimetall- oder quaternäres Ammoniumkation bedeutet.

Aus der Literatur [Tetrahedron Letters, *12*,1305—1309(1966)] ist bekannt, dass sich 3-Nitrophthalsäureanhydrid bei einer Temperatur von 110°C mit Natriumizid in Gegenwart eines inerten organischen Lösungsmittels in das 3-Azidophthalsäureanhydrid überführen lässt. Das 4-Nitrophthalsäureanhydrid und das 3-Chlorphthalsäureanhydrid lassen sich jedoch dieser Methode nicht in das entsprechende Azid überführen. Es ist daher überraschend, dass sich erfindungsgemäss sowohl die Bis-(3-Nitrophthalimide) als auch die Bis-(4-Nitrophthalimide) der Formel IV und die entsprechenden 3- und 4-Halogenverbindungen mit guten bis sehr guten Ausbeuten und zum Teil auch bei niedrigeren Temperaturen zu den entsprechenden Aziden umsetzen lassen. Eine vorherige aufwendige Auftrennung der Ausgangsprodukte in die 3- und 4-Isomeren ist daher beim erfindungsgemässen Verfahren nicht erforderlich.

$Q_1$ stellt bevorzugt die Nitrogruppe dar.

Bedeutet $M_1$ ein quaternäres Ammoniumkation, so handelt es sich z.B. um ein Tetraalkyl- oder

Benzyltrialkylammoniumkation mit je 1—12 und insbesondere 1—4 C-Atomen in den Alkylteilen, insbesondere das Tetramethyl- und Trimethylbenzylammoniumkation.

Beispiele von geeigneten Alkalimetall- und Erdalkalimetallaziden sind das Lithium-, Natrium-, Kalium-, Calcium-, Magnesium- und Bariumazid. Bevorzugt verwendet man Alkalimetallazide, vor allem das Natriumazid.

Das Azid der Formel V wird zweckmässig im Ueberschuss eingesetzt, beispielsweise in einem etwa 5—50%igen und bevorzugt in einem etwa 10—30%igen molaren Ueberschuss.

Als inerte organische Lösungsmittel kommen vor allem polare Lösungsmittel in Betracht, beispielsweise:

niedere aliphatische Alkohole, z.B. solche mit bis zu 6 C-Atomen, wie Methanol, Aethanol, Propanol, Isopropanol, Butanole und Pentanole;

Dibenzyläther und Dialkyläther mit je 1—4 C-Atomen in den Alkylteilen, wie Diäthyläther, Di-n-propyläther, Di-isopropyläther;

cyclische Aether, wie Tetrahydrofuran, Tetrahydropyran und Dioxan;

Diäthylenglykol- und Triäthylenglykoldialkyläther mit je 1—4 C-Atomen in den Alkylteilen, wie Diäthylenglykoldiäthyl- und -di-n-butyläther sowie Triäthylenglykoldimethyläther;

aliphatische und aromatische Nitrile, wie Alkylonitrile mit insgesamt 2—5 C-Atomen, z.B. Acetonitril, Propionitril, Butyronitril, Benzonitril;

ferner auch aprotische polare organische Lösungmittel der vorerwähnten Art, wie cyclische Amide, N,N-Dialkylamide von aliphatischen Monocarbonsäuren mit 1—3 C-Atomen im Säureteil, Dialkylsulfoxide, Hexamethylphosphorsäuretriamid (Hexametapol) und Tetrahydrothiophendioxide (Sulfolan).

Es können auch Gemische solcher Lösungsmittel verwendet werden.

Bevorzugte Lösungsmittel sind Dialkylsulfoxide, besonders Dimethylsulfoxid, und N,N-Dialkylamide von aliphatischen Monocarbonsäuren mit 1—3 Atomen im Säureteil, besonders N,N-Dimethylformamid und N,N-Dimethylacetamid.

Die Ausgangsprodukte der Formel II bis V sind bekannt oder können auf an sich bekannte Weise hergestellt werden. Verbeindungen der Formel IV können z.B. nach bekannten Methoden durch Umsetzen von 3- oder 4-Nitrophthalsäureanhydrid oder der entsprechenden Halogenverbindungen mit Diaminen der Formel III und anschliessende Cyclisierung der dabei gebildeten Amidcarbonsäuren erhalten werden.

Die Azidophthalsäureanhydride der Formel II sind in der US Patentschrift 3.002.003 beschrieben.

Die verbindungen der Formel Ia und Ib können auf übliche Weise isoliert und gegebenenfalls gereinigt werden, z.B. durch Abziehen des Lösungsmittels oder Einengen der Reaktionslösung der -suspension im Vakuum und anschliessendes Waschen des Reaktionsprodukts mit Wasser oder einem geeigneten inerten organischen Lösungsmittel, wie Methanol, Aethanol, Dioxan, Diäthyläther, Methylenchlorid oder Chloroform. Die Amidcarbonsäuren der Formel Ia werden im allgemeinen ohne Zwischenisolierung direkt zu den Bis-imiden der Formel Ib cyclisiert.

Die erfindungsgemässen Verbindungen der Formel Ib eignen sich zur Herstellung von unter der Einwirkung von Licht härtbaren (vernetzbaren) Stoffgemischen für photomechanische Anwendung. Derartige Stoffgemische enthalten mindestens eine polymere Verbindung und mindestens eine Verbindung der Formel Ib, wobei das Gewichtsverhältnis von polymerer Verbindung zu Verbindung der Formel Ib zweckmässig etwa 9:1 bis 1:4 und bevorzugt etwa 9:1 bis 1:1 beträgt. Als poolymere Verbindungen, die sich unter der Einwirkung von Licht, besonders UV-Licht, mit den Bis-Azidophthalimidylverbindungen der Formel Ib vernetzen bzw. härten lassen, können an sich beliebige bekannte synthetische oder natürliche Polymere eingesetzt werden, beispielsweise Polyester, Polyesteramide, Polyamide, Polyamidsäuren, Polyamidamidsäuren, Polyimide, Polyamidimide, Polyäther, Polyamine, Polyimine, Polyurethane, Polyharnstoffe, Polyurethan-harnstoffe, Polycarbonate, Polykondensate auf der Basis von Phenol-Formaldehyd, Polysacchyride, Gelatine und Polymere, die durch Homo- oder Copolymerisation von reaktiven C=C-Doppelbindungen enthaltenden Monomeren erhalten werden.

Bevorzugt sind Polyäther, insbesondere solche, die wiederkehrende Strukturelemente der Formel VI

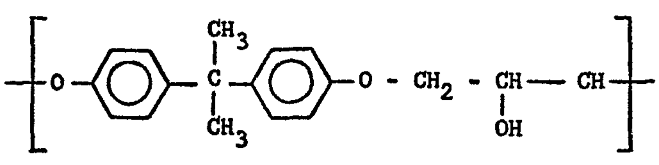

(VI)

aufweisen; Phenol-Formaldehyd-Kondensationsprodukte (Novolake), die wiederkehrende Strukturelemente der Formel VII

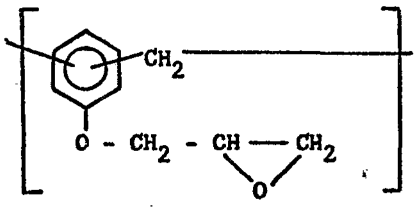

(VII)

aufweisen; Homo- oder Copolymere aus äthylenisch ungesättigten Monomeren, die gleiche oder verschiedene wiederkehrende Strukturelemente der Formel VIII

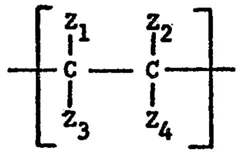

(VIII)

aufweisen, und cyclisierte Isoprenpolymere.

In Formel VIII bedeuten $Z_1$ und $Z_3$ je Wasserstoff, $Z_2$ Wasserstoff, Chlor oder Methyl und $Z_4$ Wasserstoff, Methyl, Chlor, —CN, —COOH, —$CONH_2$, Phenyl, Methylphenyl, Methoxyphenyl, Cyclohexyl, Pyridyl, Imidazolyl, Pyrrolidonyl, —COO-Alkyl mit 1—12 C-Atomen im Alkylteil, —COO-Phenyl,

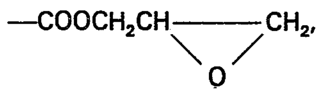

—COO-Alkylen-OH mit 1—3 C-Atomen im Alkylen,

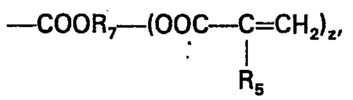

worin $R_7$ einen geradkettigen oder verzweigten gesättigten aliphatischen Rest mit 1—10 C-Atomen, $R_5$ Wasserstoff oder Methyl und z eine ganze Zahl von 1—3 bedeuten; —CO-Alkyl mit 1—4 C-Atomen im Alkyl, —OCO-Phenyl. —CO-Alkyl mit 1—3 C-Atomen im Alkyl, Alkoxy mit 1—6 C-Atomen, Phenoxy, —$CH{=}CH_2$ oder

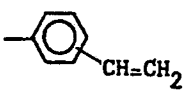

oder $Z_1$ und $Z_2$ bedeuten je Wasserstoff und $Z_3$ und $Z_4$ stellen zusammen die Gruppierung

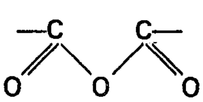

oder je —COOH oder —COO-Alkyl mit 1—6 C-Atomen im Alkyl dar.

Besonders bevorzugt sind Polyvinylchlorid, Polystyrol, Polyacrylsäure- und Polymethacrylsäurealkylester mit 1—8 C-Atomen im Alkylteil, cyclisierte Isoprenpolymere, Copolymere und Maleinsäureanhydrid und Vinyläthern oder α-Olefinen, wie Methylvinyläther oder Aethylen, sowie Polyäther mit wiederkehrenden Strukturelementen der Formel VI.

Derartige Stoffgemische eignen sich z.B. zur Herstellung von Druckplatten für das Offsetdruckverfahren, zur Herstellung von Photooffset-Lacken, für die unkonventionelle Photographie, z.B. zur Herstellung von sogenannten Vesikularbildern oder zum Anfärben von nach dem Belichten und Entwickeln schlecht sichtbaren Polymerbildern mit geeigneten Farbstoffen, wie öllösliche Farbstoffe oder, wenn das Polymere saure Gruppen, wie Carbonsäure- oder Sulfonsäuregruppen aufweist, kationische Farbstoffe. Die erwähnten Stoffgemische Finden insbesondere Anwendung als sogenannte Photoresists zur Herstellung von gedruckten Schaltungen nach an sich bekannten Methoden. Dabei wird die mit der lichtempfindlichen Schicht versehene Seite der Leiterplatte durch ein das Leiterbild aufweisendes Dia-negativ belichtet und dann entwickelt, worauf man die unbelichteten Stellen der Schicht durch Entwicklungsflüssigkeit herausholt. Die Belichtung kann mit Sonnenlicht, Kohlelichtbogen oder

Xenonlampen durchgeführt werden. Mit Vorteil wird die Belichtung mit Quecksilberhochdrucklampen vorgenommen.

Beispiel 1

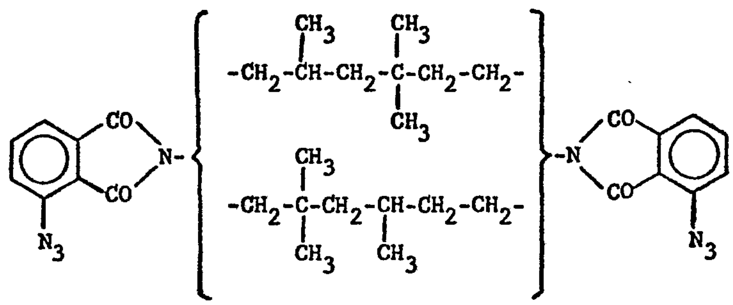

20,3 g (0,04 Mol) eines Isomerengemisches von 1,6-Bis(3-Nitrophthalsäureimidyl)-2-methyl-4-dimethylhexan und 1,6-Bis(3-Nitrophthalsäureimidyl)-2-dimethyl-4-methylhexan werden mit 7,78 g (0,104 Mol) $NaN_3$ in 100 ml N,N-Dimethylformamid 20 Stunden bei 80°C gerührt. Das Reaktionsgemisch wird bei 80°C im Vakuum eingedampft, der ölige Rückstand wird in 200 ml Wasser suspendiert und 60 Stunden intensiv gerührt. Die Suspension, die allmählich erstarrt, wird abgesaugt, mit Wasser nachgewaschen und bei 25°C über Phosphorpentoxid während 6 Tagen getrocknet. Man erhält 17,6 g (88% d.Th.) eines Isomerengemisches aus 1,6-Bis(3-Azidophthalsäureimidyl)-2-methyl-4-dimethylhexan und 1,6-Bis(3-Azidophthalsäureimidyl)-2-dimethyl-4-methylhexan. Das Produkt liegt als feinverteilte glasige Masse vor, die beim Erhitzen zerfliesst uns sich bei 180°C unter Aufschäumen zersetzt.

IR-Spektrum (KBr): 1780 $cm^{-1}$ und 1725 $cm^{-1}$ (CO—N—CO);
2120 $cm^{-1}$ ($N_3$).

Das im obigen Beispiel verwendete Isomerengemisch von 1,6-Bis(3-Nitrophthalsäureimidyl)-2-methyl-4-dimethylhexan und 1,6-Bis-(3-Nitrophthalsäureimidyl)-2-dimethyl-4-methylhexan kann wie folgt hergestellt werden:

8,1 g (0,042 Mol) 3-Nitrophthalsäureanhydrid, 3,12 g (0,02 Mol) Montamin (Isomerengemisch von 1,6-Diamino-2-methyl-4-dimethylhexan und 1,6-Diamino-2-dimethyl-4-methylhexan) und 30 ml Essigsäure werden im Autoklaven während 12 Stunden auf 130°C erhitzt. Das Reaktionsgemisch wird dann bei 80°C im Vakuum bis zur Trockne eingeengt, in 200 ml Methylenchlorid gelöst und mit gesättigter Natriumbicarbonatlösung extrahiert. Die Methylenchloridlösung wird mit wasserfreiem Natriumsulfat getrocknet und schliesslich eingedampft. Man erhält 8,7 g (86% d.Th.) Isomerengemisch von 1,6-Bis(3-Nitrophthalsäureimidyl)-2-methyl-4-dimethylhexan und -2-dimethyl-4-methylhexan in Form eines gelben, icht destillierbaren Oels.

IR-Spektrum ($CH_2Cl_2$): 1785 $cm^{-1}$ und 1725 $cm^{-1}$ (CO—N—CO);
1550 $cm^{-1}$ und 1360 $cm^{-1}$ ($NO_2$).

Beispiel 2

9,5 g (0,017 Mol) 4,4'-Bis(3-Nitrophthalsäure-imidyl)-diphenylmethan und 2,8 g (0,044 Mol) Natriumazid werden in 80 ml Dimethylsulfoxid gelöst und 14 Stunden bei 80°C gerührt. Das Reaktionsgemisch wird dann bei 80°C im Vakuum eingeengt, mit 100 ml Wasser verrührt, abgesaugt und mit 10 ml Wasser nachgewaschen. Man erhält 8,8 g (81,5% d.Th.) 4,4'-Bis(3-Azidophthalsäureimidyl)-diphenylmethan. Fp. 182°C (Zersetzung).

IR-Spektrum (KBr): 1790 $cm^{-1}$ und 1725 $cm^{-1}$ (CO—N—CO);
2130 $cm^{-1}$ ($N_3$).

Das im obigen Beispiel verwendete 4,4'-Bis(3-Nitrophthalsäureimidyl)-diphenylmethan kann wie folgt hergestellt werden:

44.36 g (0,23 Mol) 3-Nitrophthalsäureanhydrid und 21,61 g (0,109 Mol) 4,4'-Diaminodiphenylmethan werden in 300 ml Essigsäure während 5 Stunden zum Rückfluss erhitzt, dann im Vakuum eingeengt und mit 400 ml Wasser verrührt. Der entstandene gelbe kristalline Niederschlag wird abgesaugt, dann bei 40°C mit 20%iger wässriger NaOH-Lösung verrührt, mit Wasser nachgewaschen und wieder abgesaugt. Nach dem Verrühren der noch feuchten Substanz mit 200 ml Methylalkohol wird das Produkt abgesaugt und 24 Stunden bei 120°C 1,333.$10^4$ Pa getrocknet. Man erhält 54 g (90% d.Th.) 4,4'-Bis(3-Nitrophthalsäureimidyl)-diphenylmethan. Fp. 260—262°C (Zersetzung).

IR-Spektrum (KBr): 1790 $cm^{-1}$ und 1730 $cm^{-1}$ (CO—N—CO);
1550 $cm^{-1}$ und 1360 $cm^{-1}$ ($NO_2$).

Beispiel 3

22 g (0,04 Mol) 4,4'-Bis(3-Nitrophthalimidyl)-diphenyläther werden in 100 ml Dimethylsulfoxid

bei 100°C gelöst und mit 7,78 g (0,104 Mol) natriumazid versetzt. Das Reaktionsgemisch wird 16 Stunden bei 100°C gerührt und danach im Vakuum bei 100°C eingeengt. Der Rückstand wird mit 200 ml Wasser verrührt, abgesaugt und 24 Stunden im Trockenschrank bei 100°C getrocknet. Man erhält 19,2 g (88,5% d.Th.) 4,4'-(3-Azidophthalimidyl-)diphenyläther. Fp. 185°C (Zersetzung).
IR-Spektrum (KBr): 1790 $cm^{-1}$ und 1725 $cm^{-1}$ (CO—N—CO);
2130 $cm^{-1}$ ($N_3$).

Der im obigen Beispiel verwendete 4,4'-Bis(3-Nitrophthalimidyl)-diphenyläther kann wie folgt hergestellt werden:

21,86 g (0,113 Mol) 3-Nitrophthalsäureanhydrid werden in 50 ml N,N-Dimethylformamid gelöst, worauf man eine Lösung von 10,8 g (0,054 Mol) 4,4'-Diaminodiphenyläther in 50 ml N,N-Dimethylformamid zutropft. Danach gibt man 200 ml Toluol zu, erhitzt das Reaktionsgemisch zum Sieden und destilliert das entstehende Wasser azeotrop ab. Das Reaktionsgemisch wird zur Trockne eingedampft, und der Rückstand wird mit Wasser verrührt, abgesaugt und bei 100°C getrocknet. Man erhält 29,5 g (98% d.Th.) 4,4'-(3-Nitrophthalimidyl)-diphenyläther als hellgelbes Pulver; Fp. 296°C (Zersetzung).
IR-Spektrum (KBr): 1790 $cm^{-1}$ und 1730 $cm^{-1}$ (CO—N—CO);
1550 $cm^{-1}$ und 1360 $cm^{-1}$ ($NO_2$).

Beispiel 4

Ein Gemisch von 21,3 g (0,048 Mol) 1,4-Bis(3-Nitrophthalsäureimidyl)-butan und 7,2 g (0,11 Mol) Natriumazid in 225 ml N,N-Dimethylformamid wird 18 Stunden auf 80°C eritzt und nachher bei der gleichen Temperatur im Vakuum eingedampft. Der Rückstand wird mit 200 ml Wasser verrührt und mit 1 ml konzentrierter Salzsäure angesäuert. Die entstande Suspension des Produktes wird abgesaugt, mit 20 ml Wasser nachgewaschen und 24 Stunden bei 80°C im Trockenschrank getrocknet. Man erhält 20,4 g (98,8% d.Th.) 1,4-Bis(3-Azidophthalsäureimidyl)-butan; Fp.162°C (Zersetzung).
IR-Spektrum (KBr): 1775 $cm^{-1}$ und 1720 $cm^{-1}$ (CO—N—CO);
2120 $cm^{-1}$ ($N_3$).

Das im obigen Beispiel verwendete 1,4-Bis(3-Nitrophthalsäureimidyl)-butan kann wie folgt hergestellt werden:

In einem Autoklaven werden 8,1 g (0,042 Mol) 3-Nitrophthalsäureanhydrid in 15 ml Essigsäure suspendiert, mit 1,76 g (0,02 Mol) 1,4-Diaminobutan in 15 ml Toluol versetzt und 6 Stunden bei 120°C gerührt. Der entstandene dicke Niederschlag wird abgesaugt und 24 Stunden bei 120°C/ $1,333.10^4$ Pa getrocknet. Man erhält 7,8 g (90% d.Th.) 1,4-Bis-(3-Nitrophthalsäureimidyl)-butan; 245°C.
IR-Spektrum (KBr): 1780 $cm^{-1}$ und 1725 $cm^{-1}$ (CO—N—CO);
1550 $cm^{-1}$ und 1350 $cm^{-1}$ ($NO_2$).

Beispiel 5

Die Lösungen von 3,78 g (0,02 Mol) 3-Azidophthalsäureanhydrid in 50 ml Methylenchlorid und 1,98 g (0,01 Mol) 4,4'-Diaminodiphenylmethan in 50 ml Methylenchlorid werden vermischt. Nach kurzer Zeit kristallisiert die entsprechende Amidsäure aus. Diese wird nach dem Absaugen in 100 ml Dioxan suspendiert, mit 5 ml Acetanhydrid versetzt und 1 Stunde auf 85°C erhitzt. Die Lösung wird dann bis zur Trockne eingeengt, mit Wasser verrührt, mit Aethanol nachgewaschen und bei 60°C getrocknet. Man erhält 4,3 g (80% d.Th.) 4,4'-Bis(3-Azidophthalsäureimidyl)-diphenylmethan; Fp. 180°C (Zersetzung).

Beispiele 6 und 7

Analog der in den Beispielen 1 bis 4 beschriebenen Arbeitsweise werden 1,6-Bis(3-Azidophthalimidyl)-hexan und 1.10-Bis-(3-Azidophthalimidyl)-1,10-dimethyldecan hergestellt aus:

20 g (0,043 Mol), 1,6-Bis-(3-Nitrophthalimidyl)-hexan und 7,2 g (0,111 mol) Natriumazid; Ausbeute 78% d.Th., Fp. 147°C (Zersetzung) bzw.

8 g (0,014 Mol) 1,10-Bis-(3-Nitrophthalimidyl) 1,10-dimethyldecan und 2,36 g (0,035 Mol) Natriumazid; Ausbeute 80% d.Th., dickflüssiges Oel.

Beispiel 8

Mit einer Lösung von 3 g 1,4-Bis-(3-Azidophthalimidyl)-butan und 5 g eines Copolymeren aus Maleinsäureanhydrid und Aethylen ("EMA 21", Handelsprodukt der Fa. Monsanto) in 72 ml N,N-Dimethylformamid wird eine Aluminiumplatte mittels einer Schleuderzentrifuge beschichtet (300 Umdrehungen/Minute). Nach dem Trocken des Ueberzugs wird die beschichtete Platte während 1 Minute durch ein Strichnegativ mit UV-Licht belichtet (Quecksilber-Hochdruckbrenner mit vorgeschaltetem Pyrex®-Filter, Wellenlänge über 320 nm). Danach wird die belichtete Platte in 5—10%iger wässriger Natriumbicarbonat-Lösung entwickelt, wobei eine dem Strichnegativ entsprechendes Reliefbild entsteht, das gewünschtenfalls mit einem kationischen Farbstoff angefärbt werden kann.

Beispiel 9

1,35 g 4,4'-Bis-(3-Azidophthalimidyl)-diphenyläther werden in 100 ml Cyclohexanon gelöst und

dann unter Lichtausschluss mit 100 g einer 2,5%igen Lösung eines synthetischen Isopren-Polymeren ("Cariflex IR 309®", Handelsprodukt der Fa. Shell) in Chlorbenzol vermischt.

Mit dieser Lösung wird eine kupferkaschierte Epoxiplatte beschichtet, die Schicht wird bei 40°C im Vakuum getrocknet und dann durch eine Negativ mit UV-Licht ($\lambda$ über 320 nm) belichtet. Nach dem Entwickeln mit 1,1,1-Trichloräthan erscheint ein dem Negativ entsprechendes Bild.

Beispiel 10

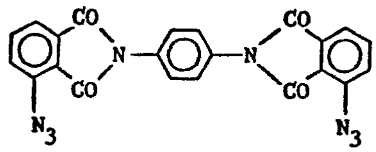

Ein Gemisch von 9,16 g (0,02 Mol) 1,4-Bis (3-nitrophthalsäureimidyl)-benzen, 2,84 g (0,044 Mol) Natriumazid und 180 ml Dimethylsulfoxid wird 24 Stunden bei 80°C gerührt. Das Reaktionsgemisch wird im Vakuum eingedampft und mit 200 ml Wasser verrührt. Die ausgefallenen Kristalle werden abgesaugt, mit 100 ml Wasser nachgewaschen und 12 Stunden bei 80°C/ 1,333.10$^4$ Pa getrocknet. Man erhält 8,8 g (98% d. Th.) 1,4-Bis(3-azidophthalsäureimidyl)-benzen; Fp. 189°C (Zersetzung).

IR-Spektrum (KBr): 1780 cm$^{-1}$ und 1725 cm$^{-1}$ (CO—N—CO)
2130 cm$^{-1}$ (—$N_3$)

Das im obigen Beispiel verwendete 1,4-Bis(3-nitrophthalsäure-imidyl)-benzen kann wie folgt hergestellt werden. Ein Gemisch von 21,2 g (0,11 Mol) 3-Nitrophthalsäureanhydrid, 5,4 g (0,05 Mol) p-Phenylendiamin, 100 ml Dimethylformamid und 200 ml Toluol wird gekocht, wobei das im Verlaufe der Reaktion entstehende Wasser azeotrop beseitigt wird. Das Reaktionsgemisch wird dann zur Trockne eingedampft, und der Rückstand mit 1000 ml Wasser verrührt, abgesaugt und 24 Stunden bei 100°C/1,333.10$^4$ Pa getrocknet. Man erhält 16,8 g (73% d.Th.) 1,4-Bis(3-nitrophthalsäureimidyl)-benzen Fp: über 250°C.

Beispiel 11

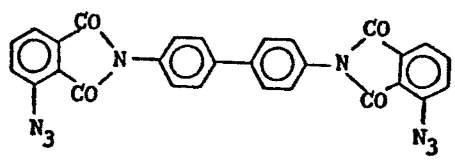

Ein Gemisch von 10,7 g (0,02 Mol) 4,4′-Bis[3-nitrophthalsäureimidyl]-diphenyl, 2,84 g (0,044 Mol) Natriumazid und 20 ml Dimethylsulfoxid werden 24 Stunden bei 80°C gerührt. Das Reaktionsgemisch wird zur Trockne eingedampft und der Rückstand mit 100 ml Wasser verrührt. Die erhaltenen Kristalle werden zweimal mit je 50 ml Wasser nachgewaschen und bei 80°C/1,333.10$^4$ Pa 24 Stunden getrocknet.

Man erhält 9,8 g (92,8% d.Th.) 4,4′-Bis[3-azidophthasäureimidyl]-diphenyl. Fp: über 250°C.

IR-Spektrum (KBr): 1775 cm$^{-1}$ und 1725 cm$^{-1}$ (CO—N—CO)
2130 cm$^{-1}$ ($N_3$)

Das im obigen beispiel verwendete 4,4′-Bis[-3-nitrophthalsäureimidyl]-diphenyl kann wie folgt hergestellt werden. Ein Gemisch von 21,2 g (0,11 Mol) 3- Nitrophthalsäureanhydrid, 8,7 g (0,05 Mol) Benzidin, 100 ml Dimethylformamid und 200 ml Toluol wird gekocht, wobei das im Verlaufe der Reaktion entstandene Wasser azeotrop beseitigt wird. Das Reaktionsgemisch wird dann zur Trockne eingedampft und der Rückstand mit 1000 ml Wasser verrührt und abgesaugt. Das noch feuchte Produkt wird mit 500 ml 10%iger Sodalösung verrührt, abgesaugt, gründlich mit Wasser gewaschen (Wasser muss zum Schluss pH 7 haben) und bei 80°C/1,333.10$^4$ Pa 24 Stunden getrocknet. Man erhält 20,2 g (75,6% d.Th.) 4,4′-Bis[3-nitrophthalsäureimidyl]-di-phenyl. Fp.: über 250°C.

Beispiel 12

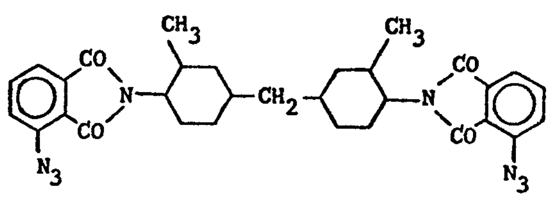

Ein Gemisch von 11,74 g (0,02 Mol) 3,3′-Dimethyl-4,4′-bis[3-nitrophthalsäureimidyl]-dicyclohexylmethan 3,64 g (0,056 Mol) Natriumazid und 180 ml Dimethylsulfoxid wurde 24 Stunden bei

80°C gerührt. Das Reaktionsgemisch wird zur Trockne eingedampft und er Rüskstand mit 100 ml Wasser verrührt, abgesaugt, dreimal mit je 50 ml Wasser nachgewaschen und über Phosphorpentoxyd 24 Stunden bei 40°C/0,1 Torr getrocknet. Man erhält 9,9 g (85,3% d.Th.) Fp.: 95—100°C (Zersetzung) 3,3'-Dimethyl-4,4'-bis[3-azidophthalsäureimidyl]-dicyclohexylmethan.

IR-Spektrum (KBr): 1780 und 1725 $cm^{-1}$ (CO—N—CO)
2130 $cm^{-1}$ ($N_3$)

Das im obigen Beispiel verwendete 3,3'-Dimethyl-4,4'-bis [3-nitrophthalsäureimidyl]-dicyclohexylmethan kann wie folgt hergestellt werden.

Ein Gemisch von 21,2 g (0,11 Mol) 3-Nitrophthalsäureanhydrid, 11,9 (0,05 Mol) 3,3'-Dimethyl-4,4'-diaminodicyclohexylmethan, 100 ml Dimethylformamid 200 ml Toluol wird gekocht, wobei das im Verlaufe der Reaktion entstandene Wasser azeotrop beseitigt wird. Das Reaktionsgemisch wird dann zur Trockne eingeengt, der Rückstand dreimal mit je 100 ml Wasser verrührt und abgesaugt. Das noch feuchte Produkt wird mit 250 ml 10%iger Sodalösung verrührt, abgesaugt, bis zur neutralen Reaktion mit Wasser gewaschen und bei 80°C/1,333.$10^4$ Pa 24 Stunden getrocknet. Man erhält 29 g (98,6% d.Th.) Fp; 125°C 3,3'-Dimethyl-4,4'-bis (3-nitrophthalsäureimidyl)-dicyclohexylmethan.

**Patentansprüche:**

1. Eine Verbindung der Formel Ia und Ib

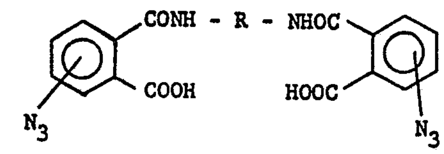

(Ia)

oder

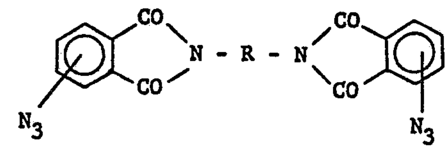

(Ib)

worin

R unsubstituiertes oder substituiertes Alkylen mit 2—18 C-Atomen, unsubstituiertes oder substituiertes Phenylen, Naphthylen, Biphenylen, Cyclohexylen, unsubstituiertes oder substituiertes Dicylohexylenmethan oder eine unsubstituierte oder substituierte Gruppierung

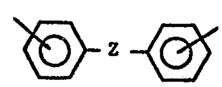

und

Z —O—, —S—, —$SO_2$—, —$CH_2$—, —CO—, —CH($CH_3$)— oder —C($CH_3$)$_2$—

bedeuten.

2. Eine Verbindung der Formel Ia oder Ib nach Anspruch 1, worin R unsubstituiertes oder durch eine oder zwei Phenylgruppen, Cycloalkylgruppen mit 5—8 C-Atomen oder Aralkylgruppen mit 7 oder 8 C-Atomen substituiertes Alkylen mit 2—12 C-Atomen, unsubstituiertes oder pro Ring durch eine Alkylgruppe mit 1—4 C-Atomen, eine —OH—, —COO—$M^+$— oder —$SO_3$—$M^+$-Gruppe substituiertes Phenylen, Naphthylen, Biphenylen, Cyclohexylen, Dicyclohexylenmethan oder

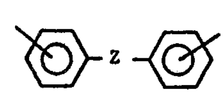

darstellt, Z die unter Formel Ia bzw. Ib angegebene Bedeutung hat, $M^+$ das Wasserstoffion, ein Alkametallkation, das Pyridiniumkation oder

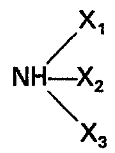

$X_1$ und $X_2$ unabhängig voneinander Wasserstoff oder Alkyl mit 1—12 C-Atomen und $X_3$ Wasserstoff, Alkyl mit 1—12 C-Atomen oder Benzyl bedeuten.

3. Eine Verbindung der Formel Ia und Ib nach Anspruch 1, worin die $N_3$-Gruppen je in ortho-Stellung zu einer Carbonyl- bzw. Carboxyl- oder Carbonamidgruppe an den Benzolring gebunden sind.

4. Eine Verbindung der Formel Ia oder Ib nach Anspruch 1, worin R unsubstituiertes geradkettiges oder verzweigtes Alkylen mit 2—12 und insbesondere 2—10 C-Atomen, unsubstituiertes Phenylen, Biphenylen, einen unsubstituierten Diphenylenäther-, Diphenylenmethan- oder Diphenylensulfonrest oder den 3,3'-Dimethyl-dicyclohexylenmethanrest bedeutet.

5. Eine Verbindung nach Anspruch 1 der Formel VI

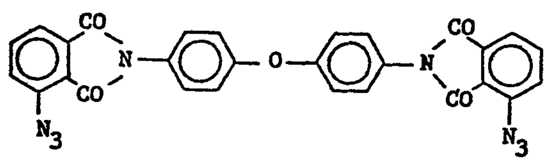

(VI)

6. Eine Verbindung nach Anspruch 1 der Formel VII

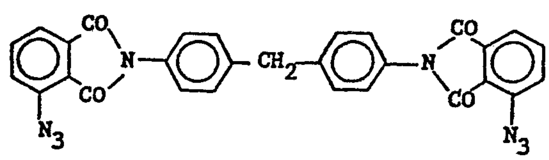

(VII)

7. Eine Verbindung nach Anspruch 1 der Formel VIII

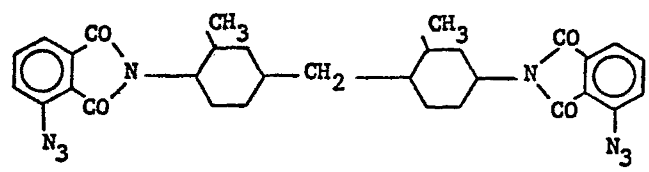

(VIII)

8. Ein Verfahren zur Herstellung einer Verbindung der Formel Ia oder Ib nach Anspruch 1, dadurch gekennzeichnet, dass man eine Verbindung der Formel II

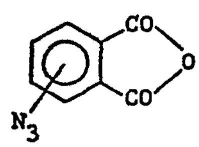

(II)

in einem Mol-Verhältnis von mindestens 1:2 mit einem Diamin der Formel III

$$H_2N—R—NH_2$$

zu einer Verbindung der Formel Ia

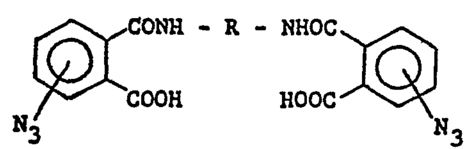

(Ia)

umsetzt, wobei R die unter Formel Ia bzw. Ib angegebene Bedeutung hat, und die Verbindung der Formel Ia gegebenenfalls anschliessend zu einer Verbindung der Formel Ib cyclisiert.

9. Ein Verfahren zur Herstellung einer Verbindung der Formel Ib nach Anspruch 1, dadurch gekennzeichnet, dass man eine Verbindung der Formel IV

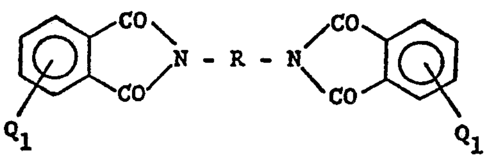

(IV),

worin
R die unter Formel Ia bzw. Ib angegebene Bedeutung hat und
$Q_1$ ein halogenatom oder die Nitrogruppe bedeutet, in einem inerten organischen Lösungsmittel bei einer Temperatur zwischen etwa 0°C und 120°C mit einem Azid der Formel V

$$M_1^{n+} (N_3^-)_n \qquad (V)$$

umsetzt, worin
n die Zahl 1 oder 2 und
$M_1$ ein Alkalimetall-, Erdhalkalimetall- oder quaternäres Ammoniumkation bedeuten.

10. Ein Verfahren nach Anspruch 9, dadurch gekennzeichnet, dass man eine Verbindung der Formel IV verwendet, worin $Q_1$ die Nitrogruppe darstellt.

11. Ein Verfahren nach Anspruch 9, dadurch gekennzeichnet, dass man als Verbindung der Formel V ein Alkalimetallazid, besonders Natriumazid, verwendet.

12. Ein Verfahren nach Anspruch 9, dadurch gekennzeichnet, dass man die Umsetzung bei einer Temperatur zwischen etwa 70 und 100°C vornimmt.

## Claims

1. A compound of the formula 1a or Ib

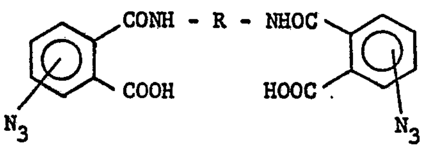

(Ia)

or

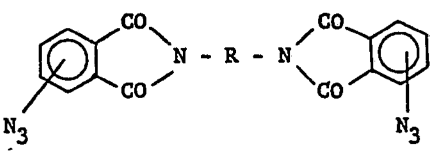

(Ib)

in which R is unsubstituted or substituted alkylene having 2—18 C atoms, unsubstituted or substituted phenylene, naphthylene, biphenylene, cyclohexylene, unsubstituted or substituted dicyclohexylmethane or an unsubstituted or substituted

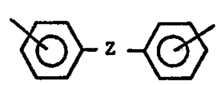

group and Z is —O—, —S—, $—SO_2—$, $—CH_2—$, —CO—, $—CH(CH_3)—$ or $—C(CH_3)_2—$

2. A compound of the formula Ia or Ib according to claim 1, in which R is alkylene having 2—12 C atoms, which is unsubstituted or substituted by one or two phenyl groups, cycloalkyl groups having 5—8 C atoms or aralkyl groups having 7 or 8 C atoms, or is phenylene, naphthylene, biphenylene, cyclohexylene, dicyclohexylmethane or

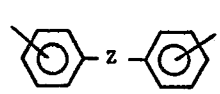

which are unsubstituted or substituted by one alkyl group having 1—4 C atoms or one —OH, $—COO—M^+$ or $—SO_3—M^+$ group per ring, Z is as defined under formula Ia or Ib, $M^+$ is a hydrogen ion, an alkali metal cation, the pyridinium cation or

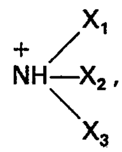

$X_1$ and $X_2$ independently of one another are hydrogen or alkyl having 1—12 C atoms and $X_3$ is hydrogen, alkyl having 1—12 C atoms or benzyl.

3. A compound of the formula Ia or Ib according to claim 1, in which the $N_3$ groups are each linked to the benzene ring in the ortho-position to a carbonyl, carboxyl or carboxamide group.

4. A compound of the formula Ia or Ib according to claim 1, in which R is unsubstituted straight-chain or branched alkylene having 2—12, and especially 2—10 C atoms, unsubstituted phenylene, biphenylene, an unsubstituted diphenylene ether, diphenylenemethane or diphenylenesulphone radical or the 3,3'-dimethyl-diclohexylenemethane radical.

5. A compound according to claim 1, of the formula VI

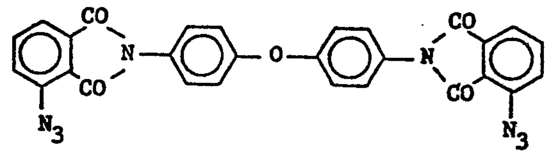

(VI)

6. A compound according to claim 1, of the formula VII

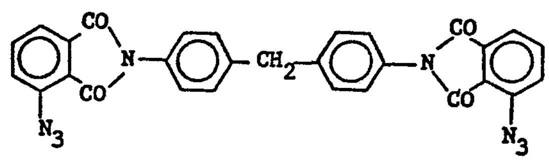

(VII)

7. A compound according to claim 1 of the formula VIII

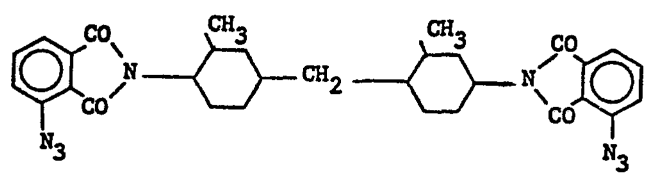

(VIII)

8. A process for the preparation of a compound of the formula Ia or Ib according to claim 1, characterized in that a compound of the formula II

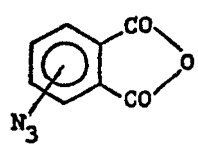

(II)

is reacted, in a molar ratio of at least 1:2, with a diamine of the formula III

$$H_2N—R—NH_2 \quad (III)$$

to give a compound of the formula Ia

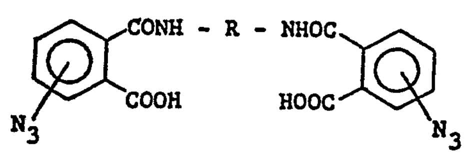

(Ia)

R being defined as under formula Ia or Ib, and, if desired, the compound of the formula Ia is subsequently cyclised to a compound of the formula Ib.

9. A process for the preparation of a compound of the formula Ib according to claim 1, characterised in that a compound of the formula IV

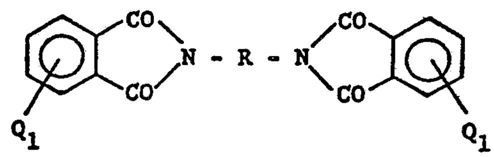

(IV)

in which R is as defined under formula Ia or Ib and $Q_1$ is a halogen atom or the nitro group, is reacted in an inert organic solvent, at a temperature between about 0°C and 120°C, with an azide of the formula V

$$M_1^{n+} (N_3^-)_n \qquad (V)$$

in which n is 1 or 2 and $M_1$ is an alkali metal cation, alkaline earth metal cation or quaternary ammonium cation.

10. A process according to claim 9, characterised in that a compound of the formula IV, in which $Q_1$ is a nitro group, is used.

11. A process according to claim 9, characterised in that an alkali metal azide, especially sodium azide, is used as the compound of the formula V.

12. A process according to claim 9, characterised in that the reaction is carried out at a temperature between about 70 and 100°C.

**Revendications**

1. Composé répondant à l'une des formules Ia et Ib

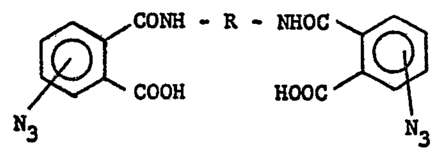

(Ia)

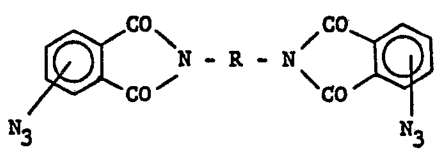

(Ib)

dans lesquelles
R représente un radical alkylène en $C_2$—$C_{18}$, substitué ou non, un radical phénylène, naphtylène, biphénylylène ou cyclohexylène, substitué ou non, un radical dicyclohexylène-méthane substitué ou non, ou un groupement, substitué ou non, répondant à la formule:

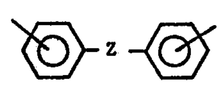

dans laquelle

Z représente —O—, —S—, —$SO_2$—, —$CH_2$—, —CO—, $-\underset{CH_3}{CH}-$ ou $-\overset{CH_3}{\underset{CH_3}{C}}-$

2. composé de formule Ia ou Ib selon la revendication I, dans lequel R représente un radical alkylène en $C_2$—$C_{12}$ non substitué ou portant un ou deux substituants pris dans l'ensemble constitué par les radicaux phényles, les radicaux cycloalkyles en $C_5$—$C_8$ et les radicaux aralkyles en $C_7$ ou $C_8$, ou un radical phénylène, naphtylène, biphénylylène, cyclohexylène, dicyclohexylène-méthane ou

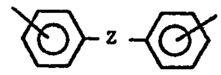

non substitué ou portant, par noyau, un radical alkyle en $C_1$—$C_4$ ou un groupe —OH, —COO—$M^+$ ou —$SO_3$—$M^+$, Z a la signification donnée à la revendication 1, $M^+$ représente l'ion hydrogène, un cation de métal alcalin, le cation pyridinium ou

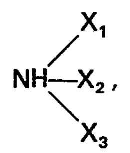

$X_1$ et $X_2$ représentent chacun, indépendamment l'un de l'autre, l'hydrogène ou un alkyle en $C_1$—$C_{12}$ et $X_3$ représente l'hydrogène, un alkyle en $C_1$—$C_{12}$ ou un benzyle.

3. Composé de formule Ia ou Ib selon la revendication 1, dans lequel chacun des groupes $N_3$ se trouve, sur son noyau benzénique, en position ortho par rapport à un groupe carbonyle ou carboxy ou carbamoyle.

4. Composé de formule Ia ou Ib selon la revendication 1, dans lequel R représente un radical alkylène en $C_2$—$C_{12}$, plus spécialement en $C_2$—$C_{10}$, linéaire ou ramifié, non substitué, un radical phénylène non substitué, un radical biphénylylène, un radical diphénylène-éther, diphénylène-méthane ou diphénylène-sulfone non substitué ou le radical diméthyl-3,3' dicyclohexylène-méthane.

5. Composé selon la revendication 1 qui répond à la formule VI

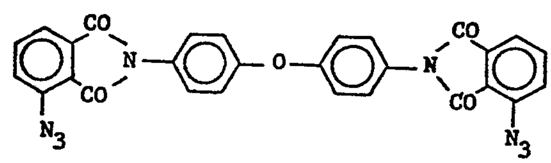

(VI)

6. Composé selon la revendication 1 qui répond à la formule VII

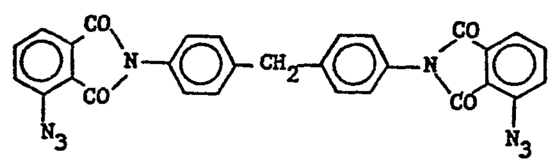

(VII)

7. Composé selon la revendication 1 qui répond à la formule VIII

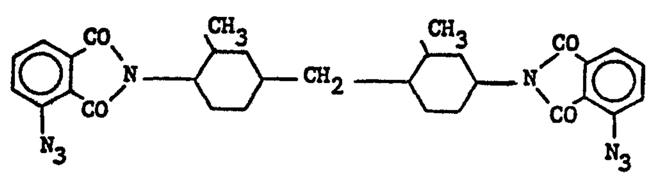

(VIII)

8. Procédé de préparation d'un composé de formule Ia ou Ib selon la revendication I, procédé caractérisé en ce qu'on fait réagir un composé de formule II

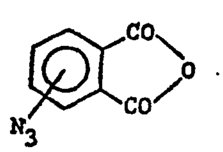

(II)

dans un rapport molaire d'au moins 1:2, avec une diamine de formule III

$$H_2N—R—NH_2 \qquad (III)$$

réaction qui conduit à un composé répondant à la formule Ia

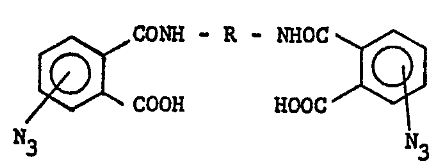

(Ia)

(le symbole R a la signification indiquée à la revendication 1), et on cyclise ensuite éventuellement le composé de formule Ia pour le convertir en un composé de formule Ib.

9. Procédé de préparation d'un composé de formule Ib selon la revendication 1, procédé caractérisé en ce qu'on fait réagir un composé répondant à la formule IV

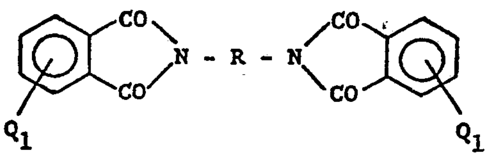

(IV),

dans laquelle
R a la signification donnée à la revendication 1 et
$Q_1$ représente un atome d'halogène ou le groupe nitro,
dans un solvant organique inerte, à une température comprise entre environ 0 et 120°C, avec un azoture répondant à la formule V

$$M_1^{n+} (N_3^-)_n \qquad (V)$$

dans laquelle
n désigne le nombre 1 ou le nombre 2 et
$M_1$ représente un cation de métal alcalin ou de métal alcalino-terreux ou un cation d'ammonium quaternaire.

10. Procédé selon la revendication 9, caractérisé en ce qu'on utilise un composé de formule IV dans lequel $Q_1$ représente le groupe nitro.

11. Procédé selon la revendication 9, caractérisé en ce qu'on utilise, comme composé de formule V, un azoture de métal alcalin, plus particulièrement l'azoture de sodium.

12. Procédé selon la revendication 9, caractérisé en ce qu'on effectue la réaction à une température comprise entre environ 70 et 100°C.